# EUROPEAN PATENT APPLICATION

(11) **EP 2 260 772 A1**
(43) Date of publication of application: **15.12.2010**
(21) Application number: 09002790.5
(22) Date of filing: 26.02.2009
(51) Int. Cl.: A61B 17/02, A61B 17/22, A61B 17/00

(54) **Endoscopic surgical instrument**

(71) Applicant: Stiftung Orthopädische Universitätsklinik Heidelberg, 69118 Heidelberg (DE)
(72) Inventor: The designation of the inventor has not yet been filed
(74) Representative: Vossius & Partner

(57) **Abstract**

The present invention relates to an endoscopic surgical instrument for the enlargement of a body cavity during a medical procedure, in particular during arthroscopy. The endoscopic surgical instrument comprises an elongated sheath insertable into the body cavity and having a longitudinal axis, a proximal end and a distal end. The endoscopic surgical instrument further comprises a rod having a proximal end and a distal end, wherein said rod is movably arranged within said sheath along the longitudinal axis thereof and a plurality of wires, each wire having a proximal end and a distal end. When the instrument is non-activated, the wires assume a longitudinally extended and radially contracted configuration, whereas, when said instrument is activated, said wires assume a longitudinal retracted and radially extended configuration.

## Description

The present invention relates to an endoscopic surgical instrument for the enlargement of a body cavity during a medical procedure.

Arthroscopy and in particular arthroscopic surgery is becoming more and more widespread. Arthroscopic surgery is a minimally invasive surgical procedure of a joint using an arthroscope. Arthroscopy is performed on joints of the knee, shoulder, elbow, wrist, ankle and hip, but also, e.g., at the intervertebral disks.

The advance of arthroscopy over traditional open surgery is that the joint does not have to be fully opened up. Instead, the endoscope and one or more surgical instruments are inserted through two or more small incisions. Accordingly, the intervention is less dramatic which reduces recovery time and may increase the rate of surgical success due to less trauma.

One problem associated with arthroscopy is the lack of space which is available to the surgeon operating on the joint. This is particularly problematic in case of the shoulder joint. It is, for example, very difficult to perform an intervention within the subacromial space of the shoulder joint or the carpal tunnel of the wrist joint by arthroscopy. Up to date, there are two ways to deal with this problem. One possibility is to apply traction to the arm which slightly removes the deltoid from the supraspinatus and the humerus. However, this may lead to damages of, e.g., the nervous system. Another option is to insert irrigation fluid into the joint to distend the joint and provide surgical space. Yet, the irrigation fluid leaks into the surrounding soft tissue causing extravasation and edema. In particularly severe cases the edema caused by the irrigation fluid may even reach to the thorax.

There is thus a need to provide a better way of making surgical space within a joint to improve and ease arthroscopy. It is in particular an object of the present invention to provide a tool which improves the visibility within a body cavity such as a joint or around a joint and to increase the field of sight. Accordingly, the present invention is directed to an endoscopic surgical instrument for the enlargement of a body cavity during a medical procedure, in particular during arthroscopy. The invention is based on the idea to provide an endoscopic tool which is insertable into the joint and which can be activated or expanded within the joint. Thus, sufficient space to provide vision to the surgeon and enough space for the surgery is created in a more gentle way thus minimizing the adverse effects of the techniques previously used.

The endoscopic surgical instrument of the present invention comprises an elongated sheath, which may be partially insertable into the body cavity, having a longitudinal axis, a proximal end and a distal end. The endoscopic surgical instrument further comprises a rod having a proximal end and a distal end, wherein said rod is movably arranged within said sheath along the longitudinal axis thereof and a plurality of wires insertable into the body cavity, each wire having a proximal end and a distal end. When the instrument is non-activated, the wires assume a longitudinally extended and radially contracted configuration, whereas, when said instrument is activated, said wires assume a longitudinal retracted and radially extended configuration.

The endoscopic surgical instrument may thus be inserted in the non-activated configuration into a body cavity such as a joint and be activated within the body cavity in order to enlarge said cavity. Said enlarged cavity may then be used to introduce further surgical instruments and to perform surgery within the body cavity. The enlargement of said body cavity in particular increases the field of sight available to the surgeon. After the intervention the endoscopic surgical instrument of the present invention may be deactivated again and withdrawn from the body cavity.

In a first preferred embodiment the proximal ends of the wires are attached to the distal end of said sheath, and the distal ends of said wires are attached to the distal end of said rod. Preferably, said rod has a rounded distal tip receiving the distal wire ends. By moving said rod together with the rounded distal tip relative to the elongated sheath, the wires, which are attached to the sheath and the rod, may be bent into a radially extended configuration. Thus, the instrument can be activated by moving the rod relative to the sheath. In order to allow for a fine adjustment of this activation said sheath preferably comprises an internal thread and said rod an external thread. In this case, the rounded distal tip which receives the wire ends can preferably freely rotate on/around said rod. Alternatively, the sheath comprises a distal ring receiving the proximal wire ends, wherein said distal ring may freely rotate around the sheath.

According to a particularly preferred alternative, the sheath further comprises an internally threaded proximal ring for cooperation with said externally threaded rod. Said proximal ring is preferably formed as a knurled screw, which is rotatable with respect to the sheath, e.g. via an axial bearing. Thus, the knurled screw can be rotated with respect to the sheath and the rod causing a displacement of the rod with respect to the sheath without any rotation of either the rod or the sheath. It is preferred that the sheath and the rod are engaged via a longitudinal guide which prevents for a relative rotation.

It is preferred that the plurality of wires are arranged about a portion of a circumference of the sheath spanning 120° to 300°, preferably 150° to 270°, most preferably 160° to 240° of said circumference. It is further preferred that the wires are arranged substantially parallel to each other. In other words, the wires are arranged substantially along said longitudinal axis of said sheath or in a helical path around said rod.

In the longitudinally extended configuration the wires are preferably curved with respect to the rod. In other words, the wires are slightly pre-bent radially outwards. Thus, a displacement of the rod with respect to the sheath forces the wires to further bend outwardly. The radius of curvature of the wires in the longitudinally extended configuration is between 0.1 m and 7 m, preferably between 0.3 m and 1 m.

It is also preferred that said rod protrudes distally from the sheath approximately by the longitudinal extension of said wires when the endoscopic surgical instrument is in the non-activated state. In other words, the plurality of wires are substantially aligned along said rod in the non-activated state. By contrast, in the activated state said rod protrudes distally from the sheath less than the longitudinal extension of said wires thus forcing the wires to bend and to extend radially from said rod. In the non-activated state, the radial distance between the wires and the centre axis of said instrument is between 0.5 mm and 5 mm, preferably between 1.5 mm and 2.5 mm. The diameter of the distal end of the endoscopic instrument in the non-activated state is between 3 mm and 10 mm, preferably between 4 mm and 6 mm. In the activated state, the radial distance between the wires and the centre axis of said instrument is between 10 mm and 25 mm, preferably between 12 mm and 17 mm. The diameter of the distal end of the endoscopic instrument in the activated state is between 15 mm and 50 mm, preferably between 25 mm and 35 mm.

It is preferred that the wires are made from a superelastic or pseudoelastic material, preferably a NiTi alloy such as Nitinol. Preferably, said wires are arranged equidistantly from each other. It is further preferred that the plurality of wires are 4 to 12 wires, preferably 6 to 10 wires, most preferably 7 or 8 wires. The wires of the endoscopic surgical instrument have a thickness in the range of from 0.4 mm to 1.0 mm, preferably of from 0.6 mm to 0.8 mm, more preferably of about 0.7 mm. The length of the wires is preferably from 30 mm to 100 mm, more preferably from 40 mm to 80 mm. Said sheath preferably has a length of from 50 mm to 300 mm, more preferably of from 60 mm to 100 mm. A preferred material for the sheath, the rod, the distal tip and/or the knurled screw is medical grade stainless steel.

According to a second preferred embodiment the plurality of wires are shape memory wires, wherein said shape memory wires are movable into and out of said distal end of said sheath by means of said rod such that, when non-activated, said wires are located at least partially within said sheath and, when activated, said wires are located substantially outside said sheath. Preferably, the proximal ends of said wires are attached to said distal end of said rod. Thus, by moving the rod with respect to the sheath in an axial direction as explained above the wires are moved in and out of said sheath. The distal wire ends of this embodiment are preferably attached to a distal tip. Since the wires are made of a shape memory material, the wires open up radially into a predetermined configuration once they are not confined by the sheath. Thus, the wires expand into the radially extended configuration. If the wires are again drawn into the sheath via said rod, the wires are forced into the radially retracted configuration.

In order to control the displacement of the rod with respect to the sheath in the axial direction and in particular to arrest the instrument in the activated state it is preferred that the rod comprises a ratchet which may interact with an unlockable latch.

The present invention is also directed to a method of enlarging a body cavity comprising the following steps: Inserting an endoscopic surgical instrument comprising a plurality of wires, in particular an instrument according to any one of the claims, into a body cavity; and activating the instrument, whereby the plurality of wires assume a radially extended configuration. Once the instrument is activated a surgical procedure may be performed within the body cavity. Afterwards, the endoscopic surgical instrument is deactivated, whereby the plurality of wires assume a radially contracted and longitudinally extended configuration; and the instrument is retreated from the body cavity.

The invention will be further elucidated with reference to the following Figures:
- Figure 1a: shows a longitudinal section of a preferred embodiment of the endoscopic surgical instrument according to the present invention in the non-activated state.
- Figure 1b: shows a cross-section through the embodiment shown in Figure 1a along line B-B.
- Figure 2: shows a longitudinal section of the embodiment shown in Figure 1 a in the activated state.
- Figure 3a: shows a longitudinal section of another preferred embodiment of the endoscopic surgical instrument according to the present invention in the non-activated state.
- Figure 3b: shows a longitudinal section of the embodiment shown in Figure 3a in the activated state.
- Figure 3c: shows a cross-section through the embodiment shown in Figure 3a along line A-A.
- Figure 4a: schematically shows a shoulder joint.
- Figure 4b: schematically shows a shoulder joint with the endoscopic instrument according to the present invention inserted into the cavity of the joint.

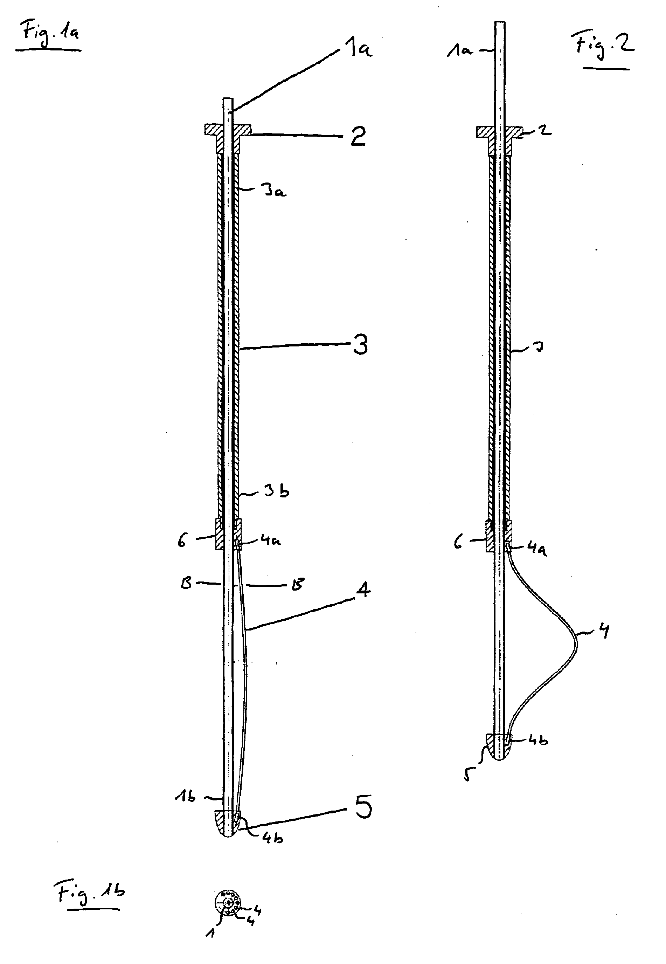

Figure 1a shows a longitudinal section through a preferred embodiment of the endoscopic surgical instrument according to the present invention. The instrument comprises a rod 1 having a proximal end 1a and a distal end 1b. The rod 1 is movably arranged within an elongated sheath 3. The elongated sheath 3 is insertable into a body cavity, e.g., a joint, and has a proximal end 3a and a distal end 3b. The endoscopic surgical instrument further comprises a plurality of wires 4 (one of which is shown in Figure 1a), wherein each wire 4 has a proximal end 4a and a distal end 4b. The proximal ends 4a of said wires 4 are attached to the distal end 3b of said sheath 3. The distal ends 4b of said wires 4 are attached to the distal end 1b of said rod 1.

In case of the embodiment shown in Figure 1a, the rod 1 has a rounded distal tip 5 receiving the distal wire ends 4b. The distal end 3b of the sheath 3 comprises a distal ring 6 which receives the proximal wire ends 4a. In the embodiment shown in Figure 1a, the sheath 3 further comprises a proximal ring 2.

In the situation shown in Figure 1 a the endoscopic surgical instrument is in the non-activated state. In other words, the wires 4 (one of which is shown in Figure 1a) assume a longitudinally extended and radially contracted configuration. Accordingly, the wires 4 are arranged substantially parallel to the longitudinal axis of the rod 1.

As can be seen in the cross-section of Figure 1b said wires 4 are arranged equidistantly from each other and substantially parallel to each other around the rod 1, wherein the plurality of wires 4 are arranged about a portion of the circumference only. In the embodiment shown in Figures 1a and 1b, the wires are arranged about a portion of the circumference spanning about 210 degrees of said circumference.

In order to activate the endoscopic surgical instrument shown in Figure 1a the rod 1 may be displaced in a longitudinal direction with respect to the sheath 3. This is shown in Figure 2, where the rod 1 has been retracted proximally with respect to the sheath 3. Accordingly, the distance between the distal ring 6 located at the distal end 3b of the sheath 3 and the rounded distal tip 5 located at the distal end 1b of the rod 1 is reduced compared to the situation shown in Figure 1a. Since the wires 4 are mounted between the rounded distal tip 5 and the distal ring 6 with their proximal and distal ends 4a and 4b being attached to the distal ring 6 and the rounded distal tip 5, respectively, the wires are forced to bend into a longitudinal retracted and radially extended configuration as can be seen in Figure 2. This corresponds to the activated state of the endoscopic surgical instrument.

In order to allow for a fine-tuning the rod 1 and the sheath 3 are engaged with each other via a threading. Preferably, said sheath 3 comprises an internal thread and said rod 1 comprises an external thread. The internal thread may be located at the distal ring 6 for cooperation with the externally threaded rod 1. Alternatively or in addition, the proximal ring 2 may comprise an internal thread. Thus, by rotating the rod 1 with respect to the sheath 3 the rod 1 may be displaced proximally and/or distally.

In order to prevent for a winding of the wires 4 when the rod 1 is rotated around its longitudinal axis, the rounded distal tip 5 is preferably able to freely rotate around the rod 1. Thus, a rotation of the rod 1 changes the distance between the rounded distal tip 5 and the distal ring 6 without affecting the orientational configuration of the wires 4. It is preferred to realize the proximal ring 2 as a knurled screw, which is rotatable with respect to the sheath 3 and the rod 1, e.g. via an axial bearing connecting the knurled screw with the sheath. Turning the knurled screw, e.g. clockwise, thus displaces the rod 1 proximally with respect to the sheath bending the wires into the extended configuration shown in Figure 2. Turning the knurled screw, e.g. counterclockwise, allows for a distal displacement of the rod due to the tension within the bent wires. Preferably, the inside of the sheath and the outside of the rod comprise a guide or track which ensures a mere longitudinal respective displacement without relative rotation of rod and sheath.

In another embodiment of the present invention the endoscopic surgical instrument makes use of shape-memory wires in order to switch from a non-activated into an activated state. In this embodiment, the wires 4 are made of a shape-memory material such as Nitinol which undergoes a phase change at a certain temperature. Accordingly, the endoscopic surgical instrument may be activated from a state shown in Figure 1a to a state shown in Figure 2 by simply changing the temperature. For this purpose, the shape-memory wires 4 are preferably movable into and out of the distal end 3b of said sheath 3. The distal end 3b and/or the distal ring 6 therefore preferably comprises recesses or bores which can partially accommodate the wires 4 in a sliding manner.

The skilled person will understand that those two embodiments may be combined such that the wires are moveable into and out of the sheath. In the non-activated configuration the wires are confined by said sheath. If the sheath is withdrawn the wires expand into a predetermined state, in which the wires open up into a radially extended configuration.

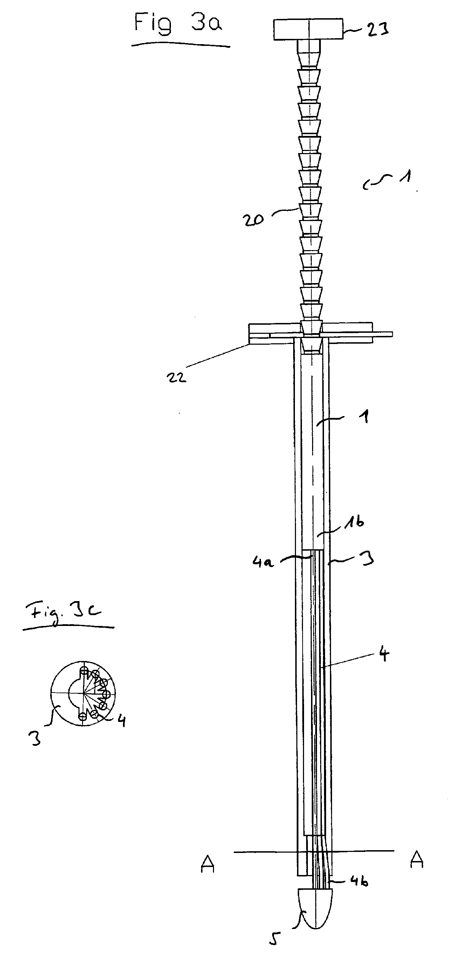

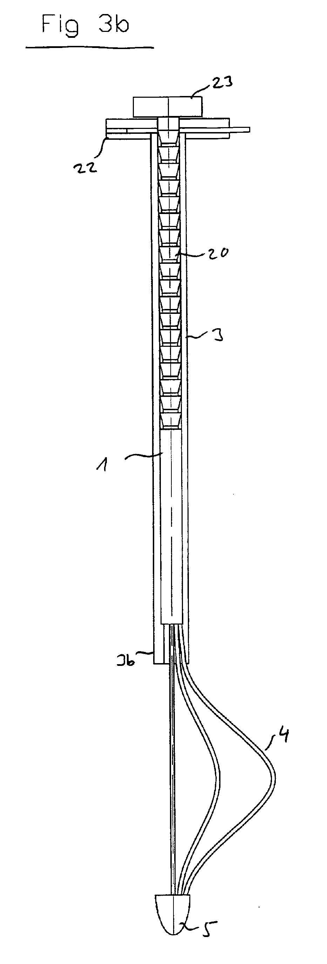

Another embodiment which utilizes wires made from a shape-memory material is shown in Figures 3a, 3b and 3c. Figure 3a shows the embodiment in the non-activated state with the rod 1 being retracted proximally with respect to the sheath 3. According to this embodiment, the proximal ends 4a of wires 4 are attached to the distal end 1b of rod 1. There is preferably no direct connection between the wires 4 and the sheath 3 present. Thus, the wires 4 are merely pushed and pulled by the rod 1, the radial expansion being only caused by the fact, that the wires 4 are made of a shape-memory material and thus "remember" a shape impressed to them before mounting of the instrument. The wires 4 are thus under tension and merely remain within the radially retracted configuration due to the surrounding sheath 3. If the sheath is withdrawn by moving the rod 1 distally as indicated in Figure 3b, the wires 4 can return to their predetermined "memorized" shape, i.e. into the radially extended shape. Several wires 4 thus form an umbrella-like shape or span a partial spherical surface.

Instead of the knurled screw 2 shown in Figures 1 and 2, the rod 1 of the embodiment shown in Figure 3a comprises a ratchet 20 which interacts with an unlockable latch 22. If the rod 1 is displaced downwards, i.e. distally with respect to the sheath 3, by means of handle 23, the ratchet 20 slidably passes the latch 22, which prevents the rod 1 from retracting proximally. Thus, the wires 4 are pushed out of the sheath 3, where they expand radially into their predetermined "memorized" shape shown in Figure 3b and the surgical instrument is maintained in its activated state by means of said ratchet 20. In order to deactivate the instrument, the latch 22 has to be unlocked which allows for a sliding displacement of the rod 1 proximally with respect to the sheath 3. Deactivating the instrument pulls the wires 4 back into the sheath 3 thereby forcing them into a longitudinally expanded state as shown in Figure 3a.

As can be seen in the cross-section of Figure 3c said wires 4 are arranged equidistantly from each other and substantially parallel to each other around the rod 1, wherein the plurality of wires 4 are arranged about a portion of the circumference only. In the embodiment shown in Figures 3a to 3c, the wires are arranged about a portion of the circumference spanning about 180 degrees of said circumference.

Evidently, the embodiment shown in Figure 3 with the proximal ends 4a of wires 4 being attached to the distal end 1b of rod 1 may alternatively utilize the knurled screw described with respect to the embodiment shown in Figure 1: Even a combination of ratchet and knurled screw is possible, where the ratchet is used for fast movement of the rod and the knurled screw provides the functionality of a fine adjustment.

Alternatively, the knurled screw of the embodiment shown in Figure 1 with the proximal ends 4a of said wires 4 being attached to the distal end 3b of said sheath 3 may be replaced by a ratchet as described above.

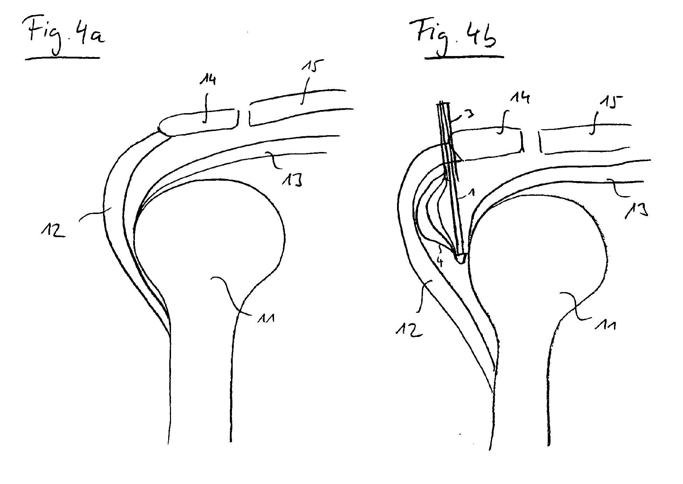

As sketched in Figures 4a and 4b, this umbrella-like shape of the wires in the extended configuration is adapted to create space in a body cavity such as a joint. Figure 4a shows a schematic representation of the human shoulder joint with humerus 11, deltoid 12, supraspinatus 13, acromion 14 and clavicle 15. As can be seen, the space between supraspinatus 13 and deltoid 12 is rather small and does neither provide much space for surgery nor sufficient vision to easily perform surgery. Accordingly, the present invention suggests to insert the endoscopic instrument comprising a sheath 3, a rod 1 and wires 4 into the cavity between supraspinatus 13 and deltoid 12 as schematically shown in Figure 4b. The instrument is then activated and the wires are expanded into the radially extended configuration as shown in Figure 4b. Due to the umbrella-like shape of the expanded wires 4 the deltoid 12 is displaced from and hold at a distance from the head of humerus 11 and the supraspinatus 13. Thus, the space available within the joint is increased.

As can be seen in Figure 4b, the distal portion of the rod 1 of the embodiment shown in Figures 1 and 2 may take away some of the newly created space and/or block the vision. It is thus apparent that the embodiment shown in Figure 3 is even more preferred since in this embodiment no central rod can block the vision.

Although the dimensions of the preferred embodiment shown in Figure 1a are optimized for an arthroscopy of the shoulder joint, the endoscopic surgical instrument of the present invention is generally applicable for the enlargement of any body cavity during a medical procedure. In particular, the endoscopical surgical instrument of the present invention may be used during arthroscopy of all major joints such as the joints of the knee, the elbow, the wrist (e.g. splitting of the carpal tunnel), the ankle, or the hip. The endoscopical surgical instrument of the present invention may further be used during operation of the intervertebral disks. The skilled person will understand that the dimensions shown in Figure 1a will have to be adapted accordingly. If the endoscopical surgical instrument of the present invention is, for example, used for arthroscopy of the hip joint, it is preferred that the wires 4 are about 10 to 20 mm longer than in case of the shoulder joint. Accordingly, the length of the wires is preferably from 40 mm to 120 mm, more preferably from 50 mm to 100 mm should the instrument be used in the hip joint. Of course, the other dimensions should then be adapted accordingly. For example, the wires of the endoscopic surgical instrument would preferably have a thickness in the range of from 0.5 mm to 1.2 mm, preferably of from 0.75 mm to 1 mm.

It should furthermore be evident that features shown in different embodiments may be combined. For example, the embodiment shown in Figure 1 may utilize a ratchet instead of or in addition to the knurled screw, while the embodiment shown in Figure 3 may also comprise the knurled screw instead of or in addition to the ratchet. While it is particularly preferred to use shape-memory wires in the embodiment shown in Figure 3, shape-memory wires may also be utilized in the embodiment of Figure 1.

The endoscopic surgical instrument of the present invention will generally facilitate arthroscopy. The instrument of the present invention can enlarge a body cavity to improve the visibility within said cavity and to enlarge the field of sight available to the surgeon. By use of said instrument surgical space can be made much easier and less harmfully than by techniques known in the art. Accordingly, trauma and risk associated with arthroscopy can be minimized. The endoscopic surgical instrument of the present invention is simple to handle and can be easily introduced through a standard incision typically made during arthroscopy.

## Claims

1. An endoscopic surgical instrument for the enlargement of a body cavity during a medical procedure comprising:
- an elongated sheath (3) having a longitudinal axis, a proximal end (3a), and a distal end (3b),
- a rod (1) having a proximal end (1a) and a distal end (1b), wherein said rod is movably arranged within said sheath (3) along the longitudinal axis thereof, and
- a plurality of wires (4) insertable into the body cavity, each having a proximal end (4a) and a distal end (4b),
wherein, when said instrument is non-activated, the wires (4) assume a longitudinally extended and radially contracted configuration and, when said instrument is activated, said wires (4) assume a longitudinally retracted and radially extended configuration.

2. The endoscopic surgical instrument according to claim 1, wherein the proximal ends (4a) of said wires (4) are attached to the distal end (3b) of said sheath (3), and the distal ends (4b) of said wires are attached to the distal end (1b) of said rod (1).

3. The endoscopic surgical instrument according to claim 2, wherein said rod has a rounded distal tip (5) receiving the distal wire ends (4b).

4. The endoscopic surgical instrument according to any one of claims 1 to 3, wherein said sheath comprises an internal thread and said rod an external thread.

5. The endoscopic surgical instrument according to any one of claims 2 to 4, wherein the sheath comprises a distal ring (6) receiving the proximal wire ends (4a).

6. The endoscopic surgical instrument according to any one of claims 1 to 5, wherein said rod comprises an external thread and said sheath comprises an internally threaded proximal ring (2) for cooperation with said externally threaded rod.

7. The endoscopic surgical instrument according to claim 6, wherein said proximal ring (2) is formed as a knurled screw, which is rotatable with respect to the sheath, e.g. via an axial bearing.

8. The endoscopic surgical instrument according to any one of claims 1 to 7, wherein the plurality of wires (4) are arranged about a portion of a circumference of the sheath (3) spanning 120° to 300°, preferably 150° to 270°, most preferably 160° to 240° of said circumference.

9. The endoscopic surgical instrument according to any one of claims 1 to 8, wherein the wires (4) are arranged substantially along said longitudinal axis of said sheath or in a helical path around said rod.

10. The endoscopic surgical instrument according to any one of claims 1 to 9, wherein the wires (4) in the longitudinally extended configuration are curved with respect to the rod.

11. The endoscopic surgical instrument according to claim 10, wherein the radius of curvature of the wires (4) in the longitudinally extended configuration is between 0.1 m and 7 m, preferably between 0.3 m and 1 m.

12. The endoscopic surgical instrument according to any one of claims 2 to 11, wherein, when in the non-activated state, said rod (1) distally protrudes from the sheath (3) approximately by the longitudinal extension of said wires (4).

13. The endoscopic surgical instrument according to any one of claims 2 to 12, wherein said wires are made from a superelastic or pseudoelastic material, preferably a NiTi alloy such as Nitinol.

14. The endoscopic surgical instrument according to any one of claims 1 to 13, wherein said plurality of wires (4) are shape-memory wires, and wherein said shape-memory wires are moveable into and out of said distal end (3b) of said sheath (3) by means of said rod such that, when non-activated, said wires are located at least partially within said sheath and, when activated, said wires are located substantially outside said sheath.

15. The endoscopic surgical instrument according to claim 14, wherein the proximal ends (4a) of said wires (4) are attached to said distal end (1b) of said rod (1).

16. The endoscopic surgical instrument according to any one of claims 1 to 15, wherein the plurality of wires are 4 to 12 wires, preferably from 6 to 10 wires, most preferably 7 or 8 wires.

17. The endoscopic surgical instrument according to any one of claims 1 to 16, wherein said rod comprises a ratchet.
